# EUROPEAN PATENT SPECIFICATION

(11) **EP 1 261 646 B1**
(45) Date of publication and mention of the grant of the patent: **21.11.2007**
(21) Application number: 01919260.8
(22) Date of filing: 06.02.2001
(51) Int. Cl.: C08B 37/08, A61K 9/36

(54) **GELS OF HYALURONIC ACID CROSS-LINKED WITH BI-FUNCTIONAL L-AMINOACIDS OR L-AMINOESTERS OR MIXTURES THEREOF**
GELE AUS HYALURONSÄURE, VERNETZT MIT BIFUNKTIONELLEN L-AMINOSÄUREN, L-AMINOSÄUREESTERN ODER DEREN MISCHUNGEN
GELS D'ACIDE HYALURONIQUE RETICULE A DES L-AMINOACIDES OU A DES L-AMINOESTERS BIFONCTIONNELS OU A DES MELANGES DE CEUX-CI

(30) Priority: 08.02.2000 IT FI20020020
(43) Date of publication of application: 04.12.2002
(73) Proprietor: Biosphere S.p.A., 47023 Cesena (Forli') (IT)
(72) Inventor: FRATINI, Luigi, I-50142 Firenze (IT); MELDOLI, Maurizio, I-47023 Cesena (IT)
(74) Representative: Gervasi, Gemma
(86) International application number: PCT/EP2001/001239
(87) International publication number: WO 2001/058961

(56) References cited:
- JP-A- 7 102 002
- US-A- 5 760 200
- US-A- 5 856 299
- TOMIHATA K ET AL: "CROSSLINKING OF HYALURONIC ACID WITH WATER-SOLUBLE CARBODIIMIDE" JOURNAL OF BIOMEDICAL MATERIALS RESEARCH,WILEY, NEW YORK, NY,US, vol. 37, no. 2, November 1997 (1997-11), pages 243-251, XP000965636 ISSN: 0021-9304 cited in the application
- BULPITT P ET AL: "NEW STRATEGY FOR CHEMICAL MODIFICATION OF HYALURONIC ACID: PREPARATION OF FUNCTIONALIZED DERIVATIVES AND THEIR USE IN THE FORMATION OF NOVEL BIOCOMPATIBLE HYDROGELS" JOURNAL OF BIOMEDICAL MATERIALS RESEARCH,WILEY, NEW YORK, NY,US, vol. 47, no. 2, 1999, pages 152-169, XP000913609 ISSN: 0021-9304

## Description

### Field of the invention

The present invention refers to gels insoluble in water consisting of hyaluronic acid cross-linked with bi-functional L-aminoacids, L-aminoesters or their mixtures, to a process for their preparation and to their use in the pharmaceutical, cosmetic and medical fields.

### State of the art

Hyaluronic acid is a mucopolysaccharide consisting of alternated units of D-glucuronic acid and N-acetyl-glucosamine, bound together by β1-3 and β1-4 bindings (1).

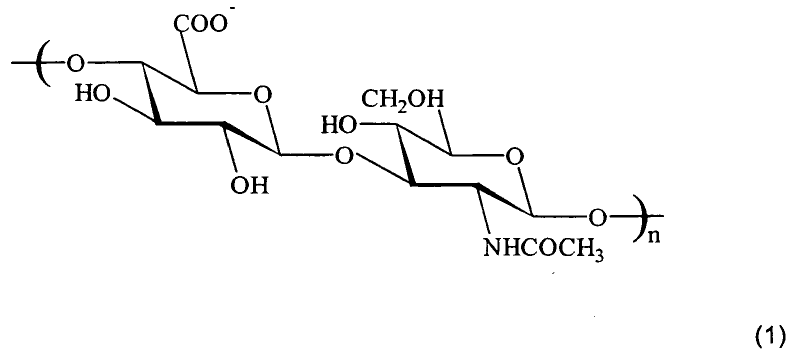

Hyaluronic acid is found in nature in the synovial liquid of articular joints, in the vitreous humor of eyes, in the umbilical cordon and in the connective tissues; can be obtained by extraction from animal tissues like cockscombs or umbilical cordons, or can be recovered from the fermenting broths of specific Streptococci.

The development of biotechnology allowed the optimisation and improvement of the last described method of production of hyaluronic acid (recovering from fermentation broths) and is nowadays the one considered the most simple and rewarding.

The essential role played by hyaluronic acid in the human body is due to the peculiar viscoelastic, lubricant and hydrophilic characteristics of its aqueous solutions.

The applications of hyaluronic acid in various fields, surgical, pharmacological or more generally biomedical, are widely described in literature, see for example: Balazs et al. "Hyaluronan Biomaterials: Medical Applications", Handbook of Biomaterials and Applications, ed. DL Wise et al., 1995, 2719-2741; US - 5,559,104, 1996; Pape, Balazs, Ophthalmology, 87, No. 7, 1980; Iwata, Clin. Orthop., 289, 285-291; 1993; US - 5,128,326; US - 4,500,676; US - 5,840,046; US - 5,795, 584; US - 6,010,692; US - 5,658,331.

Moreover a wide literature describes various compounds obtained by cross-linking hyaluronic acid with, for example, formaldehyde (Balazs, U.S. Pat. 4,713,448, 1987), divinyl sulphone (Balazs, U.S. Pat. 4,582,865, 1986), aziridine, alcohols (Della Valle, U.S. Pat. 4,851,521, 1989) and mono-functional aminoacids (Hamilton, U.S. Pat. 4,937,270, 1990).

As it can be seen from the above said, hyaluronic acid can be chemically modified in order to modify its characteristics and obtain products suitable for various applications.

It is therefore evident the importance of making available new compounds capable of widening and improving the use of hyaluronic acid in the known or in new fields.

### Detailed description of the invention

The present invention refers to gels insoluble in water prepared by cross-linking hyaluronic acid with bi-functional L-aminoacids or L-aminoesters or their mixtures.

The introduction of little biocompatible molecules as the α L-aminoacids in the hyaluronic acid chains, under particular reaction conditions, allowed the preparation of gels having the characteristic uncoloured and transparent appearance and high biocompatible and viscoelastic properties.

The products according to the present invention can be prepared in an organic solvent such as dimethylformamide (DMF) and dimethylsulphoxide (DMSO) or in water in the presence of carbodiimide according to a known process (see for example Tomihata, J. Biomed. Mater. Res., 1997, 37(2), 243-251; Danishefsky, Carbohydrate Res., 1971, 16, 199-205).

This reaction is carried out in two subsequent steps: the first step concerns the activation of hyaluronic acid, and the second one the formation of bonds between hyaluronic acid and the cross-linking agent.

During the activation step the carboxylic groups of hyaluronic acid sodium salt react with an activating compound, thus forming a novel chemical product having an increased electrophilic character of the carboxylic groups.

In the following step a cross-linking agent is added, this agent comprises two nucleophilic functions able to bind the activated carboxylic groups, making thus cross-links, i.e. bridging bonds between the hyaluronic acid molecules. Moreover, the good exiting properties of the activating agent favour the reaction.

The present activating agents are those commonly used in the literature to this aim, and in particular the water soluble carbodiimides; according to the present invention the N-3-dimethylamino-propylethylcarbodiimide hydrochloride is particularly preferred.

According to the present invention the cross-linking agents are bi-functional α L-aminoacids i.e. having a second functional group besides the aminoacidic group - or L-aminoesters or mixtures thereof. Particularly preferred are L-lysine, L-serine, L-lysine ethylester di-hydrochloride and L-serine methylester hydrochloride or mixtures thereof.

The use of aminoesters instead of aminoacids allows the protection of the carboxylic functions of aminoacids in relation to a possible activation and involvement in secondary reactions.

The whole preparation process is carried out as described herein after.

The reaction is carried out by using a glass reactor equipped with a stirring system and a temperature controller. The hyaluronic acid sodium salt commercially available is dissolved in water in a concentration comprised between 0.5 and 2.5% (w/w) following to the characteristics in the final product. For example, in order to yield compact and thick gels high concentrations are needed, for example concentrations of 2-2.5%; whereas at concentrations comprised between 0.5 and 1 % fluid gels are obtainable.

The temperature is an essential condition to obtain the products of the invention, and it must be comprised between 0°C and 25°C, and preferably between 0°C and 10°C.

The reaction mixture is then acidified by adding a diluted acid, such as hydrochoric acid 0.5-1 M, until a pH value comprised between 3 and 6, preferably 5, is reached. An activating agent able to activate the carboxylic groups of hyaluronic acid toward cross-linking, is then added. The activating agent is preferably added in a quantity comprised between 0.2 and 2 equivalents per equivalent of monomeric unit in the starting hyaluronic acid.

In succession the cross-linking agent is added, in a quantity preferably comprised between 0.1 and 2 equivalents per equivalent of monomeric unit of the starting hyaluronic acid.

Following to the additions above, the reaction mixture is maintained under stirring for a time comprised between 5 minutes and 48 hours, and preferably between 15 minutes and 5 hours.

Once the reaction is completed, a volume of the solution of NaCl 1M is added, the mixture is maintained under stirring for some minutes, then a purification is carried out according to known methods, such as dialysis and/or precipitation with organic solvent and/or under vacuum evaporation and/or freeze drying.

The reaction may be carried out in an organic solvent, such as DMSO or DMF, or in a mixture water/organic solvent in different ratios; the use of water as the reaction solvent is certainly preferred but, sometimes, it is necessary to use organic solvents for specific applications of the present products.

So the reaction is carried out, by adding in succession a solution in the organic solvent of the activating agent, preferably 2-chloro-1-methylpyridine iodide, and a suspension of the cross-linking aminoacid, to a solution of the hyaluronic acid salt of tetrabutylammonium in the organic solvent or in a mixture of the organic solvent and water, in the presence of triethylamine and under stirring, maintaining the temperature lower than 5°C.

The so obtained mixture is maintained under stirring for a time comprised between 5 minutes and 48 hours, and preferably between 15 minutes and 5 hours. The product is then recovered and purified according to the above mentioned methods.

The solid product may be dissolved again in water or in physiologic solution, in different concentrations so to obtain viscous solutions, gels, thin films, etc.

In the following examples both preparation procedures are illustrated.

The ratios hyaluronic acid/activating agent/cross-linking agent selected for the synthesis depend on the desired cross-linking degree and the viscoelastic characteristics.

The characteristics of the final product are affected by the type of starting hyaluronic acid. As a matter of fact, it is evident that, under the same conditions, a hyaluronic acid having higher molecular weight produces a more viscous and compact gel with respect to that obtainable starting from a hyaluronic acid having a lower molecular weight.

According to a preferred embodiment of the invention, a hyaluronic acid having a molecular weight comprised between 100,000 and 2,000,000 is used, and the final products obtained have a molecular weight comprised between 200,000 and 2,500,000.

The final products show a cross-linking degree comprised between 10 and 40% and an intrinsic viscosity comprised between 300 and 1,500 mg/l.

The activating agent is preferably a carbodiimide soluble in water, in particular the N-3-dimethylamino-propylethylcarbodiimide hydrochloride.

The cross-linking agent is preferably L-lysine or L-serine or esters thereof, preferably ethyl or methyl esters.

The invention will be better understood in view of the following examples.

### EXAMPLE 1

1 g of hyaluronic acid sodium salt (2.5 mmol) are dissolved in 80 ml of demineralized water. The temperature is maintained at 20°C by means of a thermostatic bath and the pH value is brought to 5 by addition of HCl 0.75 M. 0.58 g (1.2 eq) of N-3-dimethylamino-propylethylcarbodiimide hydrochloride and 0.44 g (1.2 eq) of L-lysine are added.

After 2 hours 80 ml of NaCl solution 1 M are added, and the solution is dialysed with distilled water; the product is precipitated with acetone, dissolved again in water and finally freeze-dried.

### EXAMPLE 2

1 g of hyaluronic acid sodium salt (2.5 mmol) are dissolved in 80 ml of demineralized water. The temperature is maintained at 20°C by means of a thermostatic bath and the pH value is brought to 5 by addition of HCl 0.75 M. 0.58 g (1.2 eq) of N-3-dimethylamino-propylethylcarbodiimide hydrochloride and 0.74 g (1.2 eq) of L-lysine ethyl ester di-hydrochloride are added.

After 1 hour 80 ml of NaCl solution 1 M are added, and the solution is dialysed three times, and finally freeze-dried.

### EXAMPLE 3

1 g of hyaluronic acid sodium salt (2.5 mmol) are dissolved in 80 ml of demineralized water. The temperature is maintained at 4°C by means of a thermostatic bath and the pH value is brought to 5 by addition of HCl 0.75 M. 0.48 g (1.0 eq) of N-3-dimethylamino-propylethylcarbodiimide hydrochloride and 0.62 g (1.0 eq) of L-lysine ethyl ester di-hydrochloride are added.

After 3 hour 80 ml of NaCl solution 1 M are added, and the solution is dialysed three times, and finally freeze-dried.

The ¹H-NMR analysis on the so obtained product has shown the following characteristic signals (solvent D₂O):
1.2 ppm (t, 3H, J = 10.6 Hz, CH₃-CH₂CH₂O-Lys)
1.4 ppm (m, 2H, CH₂ δ Lys)
1.6 ppm (m, 2H, CH₂ γ Lys)
1.7-1.9 ppm (m, 2H, CH₂ β Lys)
1.9-2.0 ppm (m, 3H, CH₃-CONH hyaluronic acid)
2.9 ppm (t, 2H, J = 11.2 Hz, CH₂ ε Lys)
3.0-3.9 ppm (m, CHOH hyaluronic acid)
4.0 ppm (t, 1 H, J = 9.6 Hz, CH α Lys)
4.2 ppm (q, 2H, J = 10.6 Hz, CH₃-CH₂O-Lys)

### EXAMPLE 4

1.0 g of hyaluronic acid sodium salt (2.5 mmol) are dissolved in 80 ml of demineralized water. The temperature is maintained at 4°C by means of a thermostatic bath and the pH value is brought to 5 by addition of HCl 0.75 M. 0.24 g (0.5 eq) of N-3-dimethylamino-propylethylcarbodiimide hydrochloride and 0.62 g (1.0 eq) of L-lysine ethyl ester di-hydrochloride are added.

After 3 hours 80 ml of NaCl solution 1 M are added, and the solution is dialysed three times, and finally freeze-dried.

### EXAMPLE 5

1.0 g of hyaluronic acid sodium salt (2.5 mmol) are dissolved in 80 ml of demineralized water. The temperature is maintained at 4°C by means of a thermostatic bath and the pH value is brought to 5 by addition of HCl 0.75 M.

0.24 g (0.5 eq) of N-3-dimethylamino-propylethylcarbodiimide hydrochloride and 0.62 g (1.0 eq) of L-lysine ethyl ester di-hydrochloride are added.

After 20 minutes 80 ml of NaCl solution 1 M are added, and the solution is dialysed three times, and finally freeze-dried.

### EXAMPLE 6

1.0 g of hyaluronic acid sodium salt (2.5 mmol) are dissolved in 80 ml of demineralized water. The temperature is maintained at 20°C by means of a thermostatic bath and the pH value is brought to 5 by addition of HCl 0.75 M. 0.58 g (1.2 eq) of N-3-dimethylamino-propylethylcarbodiimide hydrochloride and 0.47 g (1.2 eq) of L-serine methyl ester hydrochloride are added.

After 3 hours 80 ml of NaCl solution 1 M are added, and the solution is dialysed with distilled water; the product is then precipitated with acetone, dissolved again in water, and finally freeze-dried.

### EXAMPLE 7

1.0 g of hyaluronic acid sodium salt (2.5 mmol) are dissolved in 80 ml of demineralized water. The temperature is maintained at 5°C by means of a thermostatic bath and the pH value is brought to 5 by addition of HCl 0.75 M. 0.47 g (1.0 eq) of N-3-diethylamino-propylethylcarbodiimide hydrochloride and 0.19 g (0.5 eq) of L-serine methyl ester hydrochloride are added.

After 3 hours 80 ml of NaCl solution 1 M are added, and the solution is dialysed three times, and finally freeze-dried.

### EXAMPLE 8

1.0 g of hyaluronic acid sodium salt (2.5 mmol) are dissolved in 80 ml of demineralized water. The temperature is maintained at 0°C by means of a thermostatic bath and the pH value is brought to 5 by addition of HCl 0.75 M. 0.24 g (0.5 eq) of N-3-diethylamino-propylethylcarbodiimide hydrochloride and 0.19 g (0.5 eq) of L-serine methyl ester di-hydrochloride are added.

After 15 minutes 80 ml of NaCl solution 1 M are added, and the solution is dialysed three times, and finally freeze-dried.

### EXAMPLE 9

0.5 g of hyaluronic acid tetrabutylammonium salt are dissolved in 45 ml of DMF, under stirring and at the temperature of 5°C.

Once the salt is completely dissolved, 200 µl of triethylamine, 0.20 g of 2-chloro-1-methyl-pyridine iodide and 0.5 g of L-lysine are added.

The resulting gel is filtered, washed with water, and freeze-dried.

### EXAMPLE 10

0.3 g of hyaluronic acid tetrabutylammonium salt are dissolved in 30 ml of DMF, under stirring and at the temperature of 5°C.

Once the salt is completely dissolved, 120 ml of triethylamine, 0.12 g of 2-chloro-1-methyl-pyridine iodide and 0.21 g of L-serine are added.

The resulting gel is filtered, washed with water, and freeze-dried.

Analogously, gels of hyaluronic acid are prepared using as the cross-linking agent mixtures of L-lysine and L-serine, L-lysine ethylester di-hydrochloride and L-serine methylester hydrochloride or mixtures amnoacid/aminoacid esterified, obtaining then products having characteristics analogous to those of the products described above.

## Claims

1. A water soluble gel consisting of hyaluronic acid cross-linked with bi-functional cross-linking agents wherein said bi-functional cross-linking agents are L-aminoacids, L-aminoesters or mixture thereof.

2. The gel according to claim 1, wherein the said bi-functional cross-linking agents are L-lysine, L-serine, L-lysine ethylester di-hydrochloride, L-serine methylester hydrochloride or mixtures thereof.

3. The gel according to claims 1-2, wherein the hyaluronic acid has a molecular weight comprised between 100,000 and 2,500,000 and an intrinsic viscosity comprised between 300 and 1,500 ml/g.

4. Process for the preparation of the gel as described in claim 1, wherein:
a) the hyaluronic acid sodium salt is dissolved in water under stirring and the reaction mixture is then acidified by adding a diluted acid, until a pH value comprised between 3 and 6 is reached;
b) an activating agent and then a cross-linking agent are added to the solution under stirring;
c) once the reaction is completed, to the resulting mixture a solution of NaCl 1 M is added under stirring, then the separation and purification procedure of the so obtained product is carried out.

5. The process according to claim 4, wherein the temperature is comprised between 0°C and 25°C.

6. The process according to claim 5, wherein the temperature is comprised between 0°C and 10°C.

7. The process according to claims 4-6, wherein the activating agent is a carbodiimide soluble in water.

8. The process according to claim 7, wherein the activating agent is N-3-dimethylamino-propylethylcarbodilmide hydrochloride.

9. The process according to claim 4-8, wherein the cross-linking agent is an aminoacid as reported in claims 1 and 2.

10. The process according to claims 4-9, wherein the hyaluronic acid solution in water has a concentration comprised between 0.5 and 2.5% (w/w), the activating agent is added in a quantity comprised between 0.2 and 2 equivalents per equivalent of monomeric unit of hyaluronic acid, and the cross-linking agent is added in a quantity comprised between 0.1 and 2 equivalents per equivalent of monomeric unit of hyaluronic acid.

11. The process according to claim 10, wherein the time of reaction is comprised between 5 minutes and 48 hours.

12. The process according to claim 11, wherein the time of reaction is comprised between 15 minutes and 5 hours.

13. Use of the gel as described in claims 1-3 in surgery, in the pharmacological field or, in general, in the biomedical field.

## Patentansprüche

1. Wasserlösliches Gel bestehend aus Hyaluronsäure, die vernetzt ist mit bifunktionellen Vernetzungsmitteln, worin die bifunktionellen Vernetzungsmittel L-Aminosäuren, L-Aminoester oder Mischungen davon sind.

2. Gel gemäß Anspruch 1, worin die bifunkionellen Vernetzungsmittel L-Lysin, L-Serin, L-Lysinethylesterdihydrochlorid, L-Serinmethylester-hydrochlorid oder Mischungen davon sind.

3. Gel gemäß Ansprüchen 1 bis 2, worin die Hyaluronsäure ein Molekulargewicht zwischen 100.000 und 2.500.000 und eine intrinsische Viskosität zwischen 300 und 1.500 ml/g aufweist.

4. Verfahren zur Herstellung des Gels wie beschrieben in Anspruch 1, worin:
a) das Hyaluronsäurenatriumsalz in Wasser unter Rühren gelöst wird und die Reaktionsmischung dann durch Zugabe einer verdünnten Säure angesäuert wird, bis ein pH-Wert zwischen 3 und 6 erreicht wird;
b) ein Aktivierungsmittel und dann ein Vernetzungsmittel zur Lösung unter Rühren gegeben werden;
c) sobald die Reaktion beendet ist, zur resultierenden Mischung eine Lösung von NaCl 1 M unter Rühren gegeben wird, dann das Trennungs- und Reinigungsverfahren des so erhaltenen Produkts durchgeführt wird.

5. Verfahren gemäß Anspruch 4, worin die Temperatur zwischen 0 und 25°C ist.

6. Verfahren gemäß Anspruch 5, worin die Temperatur zwischen 0 und 10°C ist.

7. Verfahren gemäß Ansprüchen 4 bis 6, worin das Aktivierungsmittel ein in Wasser lösliches Carbodiimid ist.

8. Verfahren gemäß Anspruch 7, worin das Aktivierungsmittel N-3-Dimethylamino-propylethylcarbodiimid-hydrochlorid ist.

9. Verfahren gemäß Ansprüchen 4 bis 8, worin das Vernetzungsmittel eine Aminosäure gemäß Ansprüchen 1 und 2 ist.

10. Verfahren gemäß Ansprüchen 4 bis 9, worin die Hyaluronsäurelösung in Wasser eine Konzentration zwischen 0,5 und 2,5 % (G/G) aufweist, das Aktivierungsmittel in einer Menge zwischen 0,2 und 2 Äquivalenten pro Äquivalent monomerer Einheit von Hyaluronsäure zugegeben wird und das Vernetzungsmittel in einer Menge zwischen 0,1 und 2 Äquivalenten pro Äquivalent monomerer Einheit von Hyaluronsäure zugegeben wird.

11. Verfahren gemäß Anspruch 10, worin die Reaktionszeit zwischen 5 Minuten und 48 Stunden ist.

12. Verfahren gemäß Anspruch 11, worin die Reaktionszeit zwischen 15 Minuten und 5 Stunden ist.

13. Verwendung des Gels wie in Ansprüchen 1 bis 3 beschrieben in der Chirurgie, im pharmakologischen Gebiet oder im biomedizinischen Gebiet.

## Revendications

1. Gel hydrosoluble se composant d'acide hyaluronique réticulé à l'aide d'agents de réticulation bifonctionnels dans lequel lesdits agents de réticulation bifonctionnels sont des L-amino-acides, des L-amino-esters ou un mélange de ceux-ci.

2. Gel selon la revendication 1, dans lequel lesdits agents de réticulation bifonctionnels sont la L-lysine, la L-sérine, le dichlorhydrate d'ester éthylique de la L-lysine, le chlorhydrate d'ester méthylique de la L-sérine ou des mélanges de ceux-ci.

3. Gel selon les revendications 1 à 2, dans lequel l'acide hyaluronique présente un poids moléculaire compris entre 100 000 et 2 500 000 et une viscosité intrinsèque comprise entre 300 et 1 500 ml/g.

4. Procédé de préparation du gel selon la revendication 1, dans lequel :
a) le sel sodique de l'acide hyaluronique est dissous dans de l'eau sous agitation et le mélange réactionnel est ensuite acidifié par l'ajout d'un acide dilué jusqu'à obtenir une valeur de pH comprise entre 3 et 6 ;
b) un agent d'activation puis un agent de réticulation sont ajoutés à la solution sous agitation ;
c) une fois la réaction terminée, une solution de NaCl 1M est ajoutée sous agitation au mélange obtenu, puis la procédure de séparation et de purification du produit ainsi obtenu est réalisée.

5. Procédé selon la revendication 4, dans lequel la température est comprise entre 0°C et 25°C.

6. Procédé selon la revendication 5, dans lequel la température est comprise entre 0°C et 10°C.

7. Procédé selon les revendications 4 à 6, dans lequel l'agent d'activation est un carbodiimide hydrosoluble.

8. Procédé selon la revendication 7, dans lequel l'agent d'activation est le chlorhydrate de N-3-diméthylaminopropyléthylcarbodiimide.

9. Procédé selon les revendications 4 à 8, dans lequel l'agent de réticulation est un acide aminé selon les revendications 1 et 2.

10. Procédé selon les revendications 4 à 9, dans lequel la solution d'acide hyaluronique dans l'eau présente une concentration comprise entre 0,5 et 2,5 % (p/p), l'agent d'activation est ajouté en une quantité comprise entre 0,2 et 2 équivalents par équivalent d'unité monomère d'acide hyaluronique, et l'agent de réticulation est ajouté en une quantité comprise entre 0,1 et 2 équivalents par équivalent d'unité monomère d'acide hyaluronique.

11. Procédé selon la revendication 10, dans lequel le temps de réaction est compris entre 5 minutes et 48 heures.

12. Procédé selon la revendication 11, dans lequel le temps de réaction est compris entre 15 minutes et 5 heures.

13. Utilisation du gel selon les revendications 1 à 3 en chirurgie, dans le domaine pharmacologique ou, en général, dans le domaine biomédical.
